Europäisches Patentamt

European Patent Office

Office européen des brevets

(11) Publication number: **0 072 629**
**B1**

# EUROPEAN PATENT SPECIFICATION

(45) Date of publication of patent specification: **17.10.84**

(51) Int. Cl.³: **C 07 C 31/20, C 07 C 29/00**

(21) Application number: **82303832.8**

(22) Date of filing: **21.07.82**

(54) **Hydrogenolysis of polyols to ethylene glycol.**

(30) Priority: **22.07.81 US 287117**
**01.02.82 US 344301**

(43) Date of publication of application:
**23.02.83 Bulletin 83/08**

(45) Publication of the grant of the patent:
**17.10.84 Bulletin 84/42**

(84) Designated Contracting States:
**BE DE FR GB LU NL SE**

(56) References cited:
**DE-C-1 064 489**
**DE-C-1 126 364**
**US-A-2 965 679**
**US-A-3 396 199**

**INDUSTRIAL & ENGINEERING CHEMISTRY**
**Vol. 50, No. 8, 1958, Washington I.T. CLARK**
**"Hydrogenolysis of Sorbitol" pages 1125 to**
**1126**

(73) Proprietor: **E.I. DU PONT DE NEMOURS AND**
**COMPANY**
**1007 Market Street**
**Wilmington Delaware 19898 (US)**

(72) Inventor: **Tanikella, Murty Sundara Sita Rama**
**314 Hampton Road**
**Wilmington Delaware 19803 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

## Description

Technical field

This invention relates to a catalytic process for the hydrogenolysis of polyols to ethylene glycol.

Background information

Polyols, such as glycerol, erythritol, xylitol and sorbitol, undergo hydrogenolysis in the presence of a hydrogenation catalyst and inorganic hydroxide base to yield, primarily, glycerol, ethanediol and 1,2-propanediol. Other products may include tetritols, lactic acid, methanol, ethanol and propanol. Xylitol and sorbitol are available from biomass, such as forest and agricultural products, from which cellulose and hemicellulose can be extracted, hydrolyzed and reduced to these polyols.

Balandin A. A. et al., *Uzbekskii khimicheskii zhurnal*, Vol. 6, pp. 64—72 (1962) discloses a process for the hydrogenolysis of xylitol in the presence of 1 weight percent of calcium oxide, based on xylitol, at 200 to 230°C and 200 atm (20 MPa).

Stengel, et al., U.S. Patent 2,325,207 discloses the hydrogenolysis of carbohydrates and polyols in the presence of 5 to 7 weight percent of a catalyst comprised of a copper hydroxide and an iron and/or magnesium hydroxide, at 150 to 250°C in an alkaline environment which can be achieved by the addition of an excess of alkali, e.g., sodium hydroxide in the amount required to neutralize initial acidity and to co-precipitate the hydroxides, plus 2 to 15 weight percent, based on substrate.

Conradin, et al., U.S. Patent 2,965,679, discloses the hydrogenolysis of polyols at 200 to 300°C, 500 to 1,000 atm (50 to 101 MPa) and pH 8 to 10. Conradin, et al., U.S. Patent 3,030,429, discloses the hydrogenolysis of sorbitol and mannitol at 180 to 250°C, up to 1,000 atm (101 MPa) and pH 11 to 12.5. According to these references, the pH may be attained by the addition of calcium hydroxide.

Clark I. T., *Industrial Engineering Chemistry*, Vol. 50, pp. 1125—1126 (1958) discloses the hydrogenolysis of sorbitol in the presence of up to 3 weight percent of calcium hydroxide, based on sorbitol, at 215 to 245°C and up to 5,600 psi (39 MPa). Van Ling G. and Vlugter J. C., *Journal of Applied Chemistry*, Vol. 19, pp. 43—45 (1969) discloses the hydrogenolysis of saccharides and hexitols in the presence of up to 5 weight percent of calcium hydroxide, based on substrate, at 200 to 250°C and 100 to 300 atm (10 to 30 MPa). Similar disclosures are found in Vasyunina N. A. et al., *Doklady Akademii Nauk SSSR*, Vol. 169, No. 5, pp. 1077—1079 (1966) and U.S.S.R. Patent 422,718.

U.S. Patent 3,396,199 discloses the one-step continuous hydrogenolysis of reducible sugars to obtain a high percentage of glycerol, whereby sugar solution and hydrogen are fed continuously to a pressure reactor in the presence of a hydrogenation catalyst and a cracking additive, at a temperature of 190 to 230°C and a pressure of at least 500 psi (3.4 MPa), the cracking additive being an alkaline earth metal oxide, hydroxide or weak acid salt, the amount being 0.25 to 1.0 weight percent of CaO equivalent based on the weight of sugar the catalyst being supported nickel in amount sufficient to furnish 0.5 to 4.0 weight percent of nickel based on the weight of sugar.

Until recently, it was considered that the most economically rewarding product of the hydrogenolysis of polyols was glycerol, and that ethanediol, hereinafter referred to as ethylene glycol, could be produced more economically from other hydrocarbon sources, in particular, petroleum sources. Now, due to the scarcity and expense of petroleum, an alternative route to ethylene glycol utilizing renewable and less costly resources is desirable.

It is an object of this invention to provide a catalytic process for the hydrogenolysis of polyols which process results in high conversion of the polyols to useful products, particularly ethylene glycol. It is a particular object to provide such a process for the hydrogenolysis of pentitols and hexitols using a large amount of an inorganic hydroxide base as a promoter. It is also an object to provide a process for the hydrogenolysis of polyols, in general, which is carried out at high temperature.

Disclosure of the invention

For further comprehension of the invention and of the objects and advantages thereof, reference may be made to the following description and to the appended claims in which the various novel features of the invention are more particularly set forth.

The invention resides in the discovery that in the hydrogenolysis of polyols, i.e., compounds of the formula $C_nH_{2n+2}O_n$, such as glycerol, erythritol, xylitol, and sorbitol, the use of a large amount of an inorganic hydroxide base as a promoter and carrying out the reaction at high temperature, each enhance conversion of the polyols to useful products, particularly ethylene glycol. In addition to ethylene glycol, useful products include methanol, ethanol 1,2-propanediol, i.e., propylene glycol, and glycerol.

In particular, the invention resides in a process for the hydrogenolysis of pentitols and mixtures thereof with other polyols in which process the pentitols are contacted and reacted with hydrogen in the presence of a hydrogenation catalyst and at least 6.5 mol percent, based on the pentitols, of an inorganic hydroxide base and in said process for the hydrogenolysis of hexitols and mixtures thereof with other polyols in the presence of at least 25 mol percent, based on the hexitols, of an inorganic hydroxide base. The inorganic hydroxide base is selected from hydroxides of Group IA and IIA metals.

The amounts of catalyst discussed herein refer to the moles of the cation relative to the moles of

**0 072 629**

the specified polyols. However, use of this means of quantifying the base should not be construed as indicating that the cation moiety is more important to the reaction than the hydroxide moiety.

Further, the invention resides in a process for the hydrogenolysis of polyols to ethylene glycol in which process polyols are contacted and reacted with hydrogen in the presence of a hydrogenation catalyst and an inorganic hydroxide base at a temperature of at least 260°C, said inorganic hydroxide base being selected from hydroxides of Group IA and IIA metals.

The enhancement in the conversion of polyols to ethylene glycol as a result of the use of large amounts of an inorganic hydroxide base, i.e., more than about 6.5 mol percent in pentitols and more than about 25 mol percent in hexitols, is most pronounced when the reaction is carried out at temperatures below 260°C.

The enhancement in the conversion of polyols to ethylene glycol as a result of carrying out the reaction at greater than 260°C is most pronounced when low amounts of the inorganic hydroxide base are employed.

Xylitol and sorbitol are preferred polyols because they are readily available from cellulose and hemicellulose which are derivable from biomass. Preferably, due to economic considerations, the polyols are introduced into the reaction mixture in at least a 25 weight percent aqueous solution and, more preferably, due to convenience in handling, in a 40 weight percent aqueous solution, although the concentration is not critical. Up to about 70 weight percent results in ethylene glycol yields approximating those resulting from lower concentrations and may be more desirable because higher concentrations allow for higher throughput. They can be used separately or as a mixture. The presence in the reaction mixture, which is basic, of other substances which do not significantly adversely affect the reaction is not precluded. Further, the invention includes the described process wherein the polyols are introduced into the reaction mixture in the form of other compounds such as ketones and saccharides, which are reduced to polyols.

Hydrogen is preferably added to the reaction mixture in at least an amount stoichiometrically required to hydrogenolyze all of the polyols to ethylene glycol. Any hydrogenation catalyst may be used, e.g., nickel, palladium, or platinum. The catalyst may be free or supported. The amount of catalyst is preferably at least about 0.5 weight percent. Nickel on silica/alumina, without a promoter, can result in up to about 20 weight percent ethylene glycol; palladium on carbon, up to about 15 weight percent.

The hydroxide promoter is an inorganic hydroxide base, or mixture of such bases, selected from the hydroxides of Group IA and IIA metals, especially Ca, Sr and Ba.

Other Group IA and IIA metal hydroxides, e.g. hydroxides of Mg, Li, Na, and K may be used.

Use of these bases has been found to result in higher conversion to ethylene glycol. It will be apparent that included within the word "hydroxide" are compounds which convert to such hydroxides *in situ*, e.g., oxides.

When the reaction is carried out above 260°C at any pressure, the amount of hydroxide base is at least about 1 weight percent based on the polyols, preferably at least about 3 weight percent. The amount will vary depending upon the polyols and their relative amounts and on the bases.

Preferably, at any temperature but especially below 260°C, the amount of hydroxide base when the polyols are pentitols is at least 6.5 mol percent, more preferably at least 12 mol percent, and most preferably, at least 20 mol percent. When the polyols are hexitols, the amount is preferably at least 25 mol percent, more preferably at least 45 mol percent. Using amounts larger than the most preferred amounts does not appreciably further enhance conversion to ethylene glycol. When a mixture of polyols is used, the preferred amount will vary depending upon the relative amounts of the polyols but will not be less than 6.5 mol percent based on the pentitols or, if the mixture does not include pentitols, less than 25 mol percent based on the hexitols.

Useful temperatures for the hydrogenolysis of polyols are about 200 to 400°C. Reaction times may vary from a few minutes at about 400°C, at which some degradation may occur, to about 5 to 6 hours at about 200°C. Preferably, especially when small amounts of base are used, the reaction is carried out above 260°C, more preferably at about 275°C for about 1 hour. A wide range of pressures may be used. For example, in batch reactions, the hydrogen partial pressure, prior to heating, should be about 500 psi (3.4 MPa) to about 10,000 psi (68.9 MPa), preferably about 1000 psi (6.9 MPa) to about 8000 psi (55.2 MPa) and, more preferably, about 2000 psi (13.8 MPa) to about 7000 psi (48.3 MPa).

The reaction may be carried out in a batch, continuous or semi-continuous manner. The catalyst may be fluidized or static as with a bed of catalyst over which the reaction mixture is caused to flow.

Examples

Following are examples which are illustrative of the invention and descriptions of other reactions which are not illustrative of the invention but which are included to facilitate appreciation and understanding of the invention. All reactions were carried out substantially by the following procedure:

The polyol was mixed in water with nickel on kieselguhr or on silica/alumina and a hydroxide base and stirred until the reaction mixture appeared to be homogeneous or uniform. The mixture was then charged to a 10 cm³ pressure bomb which was placed behind a protective barrier.

3

**0 072 629**

The pressure bomb was evacuated, pressurized with hydrogen at room temperature and then heated over a period of about 30—50 minutes to the reaction temperature.

After the reaction time, the pressure bomb was allowed to cool to room temperature. The reaction products, undiluted, were filtered through a thin layer of Celite® Analytical Filter-Aid and analyzed by gas chromatography.

The conditions and results of the examples are tabulated below. Products reported are Alc (methanol and ethanol), EG (ethylene glycol), PG (propylene glycol), T (tetritols) and G (glycerol); also reported is Unr (unreacted, i.e., one of erythritol, xylitol or sorbitol) and Oth (other, e.g., higher polyols and degradation products). These are reported as weight percentages based on the total weight of all products recovered. The polyol was present in an amount of 40 weight percent based on the total weight of the reaction mixture except in the reactions reported at Tables 2 and 6. Each reported weight percent of the catalyst is based on the weight of the metal relative to the total weight of the reaction mixture. The amounts of base are reported as mol percent/weight percent. Each reported mol percent of the base is based on the moles of cation relative to the moles of polyol; each reported weight percent is based on the weight of the base relative to the weight of the polyol. Each reported reaction time is the length of time for which the reaction mixture was held at the specified reaction temperature. Each reported pressure is the hydrogen partial pressure prior to heating.

In several reactions using 40 weight percent of xylitol, 20.5 mol percent of $Ca(OH)_2$ and 0.8 weight percent of Ni on silica/alumina, at 2000 psi and 275°C for 1 hour, greater than about 90% of all materials, including products and starting materials, were recovered. It is believed that in most other reactions, product recovery was about the same, although it will be apparent that product recovery was somewhat less when high temperatures were employed. Some examples appear in more than one table.

Tables 1A and 1B illustrate the effects of base concentration and of temperature. Table 2 illustrates the effect of temperature. Table 3 illustrates the effect of base concentration. Table 4 illustrates the hydrogenolysis of a tetritol. Tables 5A and 5B illustrate the hydrogenolysis of polyols using various inorganic hydroxide bases. Table 6 illustrates the effects of polyol concentration.

TABLE 1A

Weight percent of products from the hydrogenolysis of xylitol at 2000 psi (13.8 MPa), (I): 215°C for 5 hours using 4.5 weight percent of Ni on kieselguhr; (II): 275°C for 1 hour using 0.8 weight percent of Ni on $SiO_2/Al_2O_3$; and using various amounts of $Ca(OH)_2$ in wt. %/mol %.

|  | 215°C | | | | 275°C | | | |
|---|---|---|---|---|---|---|---|---|
|  | 1 | 3 | 6 | 10 | 1 | 3 | 12 | 0 |
|  | 2.1 | 6.2 | 12.3 | 20.5 | 2.1 | 6.2 | 24.6 | 0 |
| Alc | 1 | 2 | 3 | 4 | 3 | 5 | 5 | 3 |
| EG | 12 | 28 | 37 | 43 | 30 | 35 | 42 | 20 |
| PG | 8 | 17 | 25 | 28 | 21 | 26 | 23 | 10 |
| G | 8 | 15 | 17 | 11 | 9 | 6 | 9 | 14 |
| T | 1 | 2 | 9 | 0 | 0 | 1 | 3 | 8 |
| Unr | ·61 | 21 | 3 | 8 | 24 | 8 | 8 | 13 |
| Oth | 9 | 15 | 6 | 6 | 13 | 19 | 10 | 32 |

* 4000 psi (27.6 MPa).

4

TABLE 1B

Weight percent of products from the hydrogenolysis of sorbitol at 2000 psi (13.8 MPa), (I): 215°C for 5 hours using 4.5 weight percent of Ni on kieselguhr; (II): 275°C for 1 hour using 0.8 weight percent of Ni on $SiO_2/Al_2O_3$; and using various amounts of $Ca(OH)_2$ in wt. %/mol %.

| | 215°C | | | | 275°C | | | |
|---|---|---|---|---|---|---|---|---|
| | 1 | 3 | 6 | 10 | 1 | 3 | 10 | 20 |
| | 2.5 | 7.4 | 14.8 | 24.6 | 2.5 | 7.4 | 24.6 | 49.2 |
| Alc | 1 | 1 | 3 | 4 | 7 | 9 | 6 | 8 |
| EG | 11 | 11 | 16 | 24 | 27 | 28 | 28 | 35 |
| PG | 16 | 15 | 23 | 33 | 36 | 32 | 26 | 37 |
| G | 21 | 17 | 19 | 16 | 20 | 8 | 15 | 3 |
| T | 3 | 4 | 4 | 3 | 1 | 1 | 2 | 2 |
| Unr | 32 | 28 | 14 | 0 | 0 | 3 | 6 | 1 |
| Oth | 16 | 24 | 21 | 20 | 9 | 19 | 17 | 14 |

TABLE 2

Weight percent of products from the hydrogenolysis of xylitol (25 weight percent) at 3000 psi (20.7 MPa) using 3.5 weight percent of Ni on kieselguhr and 1 weight percent of calcium oxide for two hours at various temperatures.

| | 230°C | 275°C |
|---|---|---|
| Alc | 1 | 4 |
| EG | 14 | 28 |
| PG | 9 | 19 |
| G | 9 | 15 |
| T | 1 | 1 |
| Unr | 50 | 13 |
| Oth | 16 | 20 |

TABLE 3

Weight percent of products from the hydrogenolysis of xylitol at 2000 psi (13.8 MPa), 275°C for 1 hour using 0.8 weight percent of Ni on $SiO_2/Al_2O_3$ and various amounts of NaOH.

| | NaOH (mol percent) | | | | | | | |
|---|---|---|---|---|---|---|---|---|
| | 2.1 | 6.2 | 12.3 | 20.5 | 20.6 | 38 | 61.5 | 123 |
| Alc | 3 | 5 | 5 | 5 | 4 | 7 | 11 | 7 |
| EG | 17 | 26 | 28 | 34 | 37 | 32 | 34 | 33 |
| PG | 11 | 18 | 20 | 22 | 24 | 28 | 36 | 40 |
| G | 2 | 2 | 4 | 7 | 9 | 5 | 1 | 2 |
| T | 4 | 4 | 4 | 2 | 0 | 3 | 0 | 1 |
| Unr | 37 | 19 | 20 | 13 | 10 | 1 | 0 | 0 |
| Oth | 26 | 26 | 19 | 17 | 16 | 24 | 18 | 17 |

TABLE 4

Weight percent of products from the hydrogenolysis of erythritol at 4000 psi (27.6 MPa), 275°C for 1 hour using 0.8 weight percent of Ni on $SiO_2/Al_2O_3$ and various amounts of $Ca(OH)_2$ in wt. %/mol %.

| | 10 | 20 |
|---|---|---|
| | 16.5 | 33 |
| Alc | 8 | 10 |
| EG | 37 | 32 |
| PG | 24 | 27 |
| G | 3 | 1 |
| Unr | 5 | 1 |
| Oth | 23 | 29 |

TABLE 5A

Weight percent of products from the hydrogenolysis of xylitol at 2000 psi (13.8 MPa), 275°C for 1 hour using 0.8 weight percent of Ni on $SiO_2/Al_2O_3$ and 20.5 mol percent of various hydroxide promoters.

| | Promoter cation | | | | | | | | |
|---|---|---|---|---|---|---|---|---|---|
| | Ca | Sr | Ba | Mg | Li | K | Na | Al | La |
| Alc | 5 | 5 | 8 | 6 | 3 | 5 | 5 | 4 | 8 |
| EG | 43 | 43 | 43 | 33 | 33 | 39 | 34 | 24 | 30 |
| PG | 24 | 35 | 35 | 23 | 22 | 27 | 22 | 12 | 19 |
| G | 8 | 5 | 4 | 11 | 9 | 7 | 7 | 13 | 7 |
| T | 5 | 1 | 1 | 3 | 3 | 4 | 2 | 8 | 1 |
| Unr | 3 | 2 | 0 | 6 | 16 | 5 | 13 | 3 | 1 |
| Oth | 12 | 9 | 9 | 18 | 14 | 13 | 17 | 36 | 34 |

TABLE 5B

Weight percent of products from the hydrogenolysis of sorbitol at 2000 psi (13.8 MPa), 275°C for 1 hour using 0.8 weight percent of Ni on $SiO_2/Al_2O_3$ and 24.6 mol percent of various promoters.

| | Promoter cation | | |
|---|---|---|---|
| | Ca | Na | La |
| Alc | 6 | 5 | 7 |
| EG | 28 | 20 | 19 |
| PG | 26 | 23 | 19 |
| G | 15 | 5 | 6 |
| T | 2 | 1 | 1 |
| Unr | 6 | 17 | 8 |
| Oth | 17 | 29 | 40 |

6

## TABLE 6

Weight percent of products from the hydrogenolysis of xylitol in various concentrations at 275°C for 1 hour using 2 weight percent of Ni on $SiO_2/Al_2O_3$ and 20.5 mol percent of $Ca(OH)_2$ at 4000 psi (27.6 MPa).

|     | 25% | 40% | 50% | 60% | 70% |
|-----|-----|-----|-----|-----|-----|
| Alc | 8   | 7   | 7   | 8   | 8   |
| EG  | 50  | 48  | 46  | 43  | 41  |
| PG  | 31  | 32  | 26  | 29  | 35  |
| G   | 6   | 6   | 12  | 9   | 3   |
| T   | 0   | 0   | 1   | 0   | 0   |
| Unr | 2   | 0   | 1   | 1   | 0   |
| Oth | 3   | 7   | 7   | 10  | 13  |

Best mode

The best mode for carrying out the process of the invention is illustrated by the procedure described above and, in particular, in the use of hydroxides selected from hydroxides of Ca, Ba, Sr and mixtures thereof, and about 0.8 weight percent of catalyst; when low amounts of base are employed, the reaction is carried out at or above 260°C.

Industrial applicability

The process of the invention has obvious industrial applicability as a method for producing ethylene glycol and other useful products from renewable biomass resources.

## Claims

1. Process for the hydrogenolysis of pentitols and mixtures of pentitols with other polyols to a reaction product mixture consisting essentially of a major fraction of ethylene glycol and propylene glycol and a minor fraction of glycerol, other products and unreacted starting material, in which process the pentitols or the mixtures thereof are contacted and reacted with hydrogen in the presence of a hydrogenation catalyst and at least 6.5 mol percent, based on the pentitols, of an inorganic hydroxide base selected from hydroxides of Group IA and IIA metals.

2. The process of Claim 1 wherein the pentitols include xylitol.

3. Process for the hydrogenolysis of hexitols and mixtures of hexitols with other polyols to a reaction product mixture consisting essentially of a major fraction of ethylene glycol and propylene glycol and a minor fraction of glycerol, other products and unreacted starting material, in which process the hexitols or the mixtures thereof are contacted and reacted with hydrogen in the presence of a hydrogenation catalyst and at least 25 mol percent, based on the hexitols, of an inorganic hydroxide base selected from hydroxides of Group IA and IIA metals.

4. The process of Claim 3 wherein the hexitols include sorbitol.

5. The process of any one of Claims 1 to 4 wherein the temperature is 200°C to 400°C.

6. Process for the hydrogenolysis of polyols to a reaction product mixture consisting essentially of a major fraction of ethylene glycol and propylene glycol and a minor fraction of glycerol, other products and unreacted starting material, in which process the polyols are contacted and reacted with hydrogen, at a temperature of at least 260°C, in the presence of a hydrogenation catalyst and an inorganic hydroxide base selected from hydroxides of Group IA and IIA metals.

7. The process of Claim 6 wherein the polyols are selected from the group consisting of sorbitol, xylitol, erythritol, glycerol and mixtures of any of these polyols with other polyols.

8. The process of any one of Claims 1 to 7 wherein the inorganic hydroxide base is $Ca(OH)_2$.

## Patentansprüche

1. Verfahren zur Hydrogenolyse von Pentiten und Mischungen aus Pentiten mit anderen Polyolen unter Bildung einer im wesentlichen aus einer Hauptfraktion aus Ethylenglycol und einer Nebenfraktion aus Glycerin, anderen Produkten und unumgesetztem Ausgangsmaterial bestehenden Mischung von Reaktionsprodukten, dadurch gekennzeichnet daß die Pentite oder die Mischungen derselben in Gegenwart eines Hydrierungskatalysators und von wenigstens 6,5 Mol-%, bezogen auf die Pentite, einer aus einem anorganischen Hydroxid bestehenden Base, die ausgewählt ist aus den Hydroxiden der Metalle der Gruppen IA und IIA, mit Wasserstoff in Berührung gebracht und umgesetzt werden.

**0 072 629**

2. Verfahren nach Anspruch 1, dadurch gekennzeichnet, daß die Pentite Xylit enthalten.

3. Verfahren zur Hydrogenolyse von Hexiten und Mischungen aus Hexiten mit anderen Polyolen unter Bildung einer im wesentlichen aus einer Hauptfraktion aus Ethylenglycol und Propylenglycol und einer Nebenfraktion aus Glycerin, anderen Produkten und unumgesetztem Ausgangsmaterial bestehenden Mischung von Reaktionsprodukten, dadurch gekennzeichnet daß die Hexite oder die Mischungen derselben in Gegenwart eines Hydrierungskatalysators und von wenigstens 25 Mol-%, bezogen auf die Hexite, einer aus einem anorganischen Hydroxide bestehenden Base, die ausgewählt ist aus den Hydroxiden der Metalle der Gruppen IA und IIA, mit Wasserstoff in Berührung gebracht und umgesetzt werden.

4. Verfahren nach Anspruch 1, dadurch gekennzeichnet, daß die Hexite Sorbit enthalten.

5. Verfahren nach irgendeinem der Ansprüche 1 bis 4, dadurch gekennzeichnet, daß die Temperatur 200°C bis 400°C beträgt.

6. Verfahren zur Hydrogenolyse von Polyolen unter Bildung einer im wesentlichen aus einer Hauptfraktion aus Ethylenglycol und Propylenglycol und einer Nebenfraktion aus Glycerin, anderen Produkten und unumgesetzten Ausgangsmaterial bestehenden Mischung von Reaktionsprodukten, dadurch gekennzeichnet daß die Polyole bei einer Temperatur von wenigstens 260°C in Gegenwart eines Hydrierungskatalysators und einer aus einem anorganischen Hydroxid bestehenden Base, die ausgewählt ist aus den Hydroxiden der Metalle der Gruppen IA und IIA, mit Wasserstoff in Berührung gebracht und umgesetzt werden.

7. Verfahren nach Anspruch 6, dadurch gekennzeichnet, daß die Polyole ausgewählt sind aus der Sorbit, Xylit, Erythrit, Glycerin und Mischungen beliebiger dieser Polyole mit anderen Polyolen.

8. Verfahren nach irgendeinem der Ansprüche 1 bis 7, dadurch gekennzeichnet, daß die aus einem anorganischen Hydroxid bestehenden Base Ca(OH)$_2$ ist.

**Revendications**

1. Procédé pour l'hydrogénolyse de pentitols et de mélanges de pentitols et d'autres polyols, en un mélange de produits de réaction essentiellement formé d'une fraction majoritaire d'éthylène glycol et de propylèneglycol et d'une fraction minoritaire de glycérol, d'autres produits et de matière de départ inaltérée, procédé dans lequel on met en contact les pentitols ou les mélanges de ceux-ci avec de l'hydrogène et on les fait réagir avec celui-ci en présence d'un catalyseur d'hydrogénation et d'au moins 6,5 mol %, relativement aux pentitols, d'une base du type hydroxyde minéral choisie parmi les hydroxydes de métaux des groupes IA et IIA.

2. Procédé selon la revendication 1, dans lequel les pentitols comprennent du xylitol.

3. Procédé pour l'hydrogénolyse d'hexitols et de mélanges d'hexitols et d'autres polyols, en un mélange de produits de réaction essentiellement formé d'une fraction majoritaire d'éthylèneglycol et de propylèneglycol et d'une fraction minoritaire de glycérol, d'autres produits et de matière de départ inaltérée, procédé dans lequel on met en contact les hexitols ou les mélanges de ceux-ci avec de l'hydrogène et on les fait réagir avec celui-ci en présence d'un catalyseur d'hydrogénation et d'au moins 25 mol %, relativement aux hexitols, d'une base du type hydroxyde minéral choisie parmi les hydroxydes de métaux des groupes IA et IIA.

4. Procédé selon la revendication 3, dans lequel les hexitols comprennent du sorbitol.

5. Procédé selon l'une quelconque des revendication 1 à 4, dans lequel la température est de 200 à 400°C.

6. Procédé pour l'hydrogénolyse de polyols en mélange de produit de réaction essentiellement formé d'une fraction majoritaire d'éthylèneglycol et de propylèneglycol et d'une fraction minoritaire de glycérol, d'autres produits et de matière de départ inaltérée, procédé dans lequel on met en contact les polyols avec de l'hydrogène et on les fait réagir avec celui-ci, à une température d'au moins 260°C, en présence d'un catalyseur d'hydrogénation et d'une base du type hydroxyde minéral choisie parmi les hydroxydes de métaux des groupes IA et IIA.

7. Procédé selon la revendication 6, dans lequel les polyols sont choisis dans le groupe comprenant le sorbitol, le xylitol, l'érythritol, le glycérol et les mélanges de l'un quelconque de ces polyols et d'autres polyols.

8. Procédé selon l'une quelconque des revendications 1 à 7, dans lequel la base du type hydroxyde minéral est Ca(OH)$_2$.

8